# EUROPEAN PATENT SPECIFICATION

(11) **EP 2 910 177 B1**
(45) Date of publication and mention of the grant of the patent: **31.01.2018**
(21) Application number: 15155530.7
(22) Date of filing: 18.02.2015
(51) Int. Cl.: A61B 1/00, A61B 1/04, A61B 1/06, A61B 1/05, A61B 34/00, A61B 90/98, A61B 90/00

(54) **GASTROINTESTINAL TRACT DIAGNOSIS DEVICE WITH DISPOSABLE ENDOSCOPE AND CONTROL METHOD FOR THE SAME**
MAGEN-DARM-TRAKT-DIAGNOSEVORRICHTUNG MIT EINWEGENDOSKOP UND STEUERVERFAHREN DAFÜR
DISPOSITIF DE DIAGNOSTIC DU TRACTUS GASTRO-INTESTINAL AVEC ENDOSCOPE JETABLE ET SON PROCÉDÉ DE COMMANDE

(30) Priority: 19.02.2014 TW 103105480
(43) Date of publication of application: 26.08.2015
(73) Proprietor: INSIGHT MEDICAL SOLUTIONS INC., Hsinchu City (TW)
(72) Inventor: Hong, Hei Tai, 308 Baoshan Township, Hsinchu County (TW); Tsai, Huai- Fang, Zhubei City, Hsinchu County (TW); Lu, Shih Chieh, Hsinchu City (TW); Wu, Sing, Zhongli City, Taoyuan County 320 (TW); Sung, Tze-Yun, Taoyuan City (TW); Tsai, Ping Chun, Hsinchu City (TW)
(74) Representative: Viering, Jentschura & Partner mbB Patent- und Rechtsanwälte

(56) References cited:
- US-A- 5 313 935
- US-A1- 2005 159 646
- US-A1- 2010 204 546
- US-B2- 8 562 514

## Description

### Field of the Invention

The instant disclosure is related to a gastrointestinal tract diagnosis device with disposable endoscope and control method for the same, in particular, a device and control method for the same being competent to disable the function of the endoscope, thus, forcibly rendering the disabled endoscope disposable so that public health issues of improper reusing of the endoscope can be prevented.

### Description of Related Art

Nowadays the endoscope viewing device of the gastrointestinal tract is reusable. Every time when the endoscope is used, serious cleaning and sterilization are demanded to make sure that the endoscope is clean for the next user. Otherwise infection and improper spreading of pathogen could result. As a result, threats of abnormal infection could result from the compromised sterilization of the endoscope.

Gastrointestinal viewing is basically invasive. Most medical staff hope the apparatus for gastrointestinal viewing that contacts with the living body or the inside of the living body would be disposable and only for one-time usage so that the unusual infection and spreading of the pathogen can be prevented. The examples of disposable medical equipment and demands for such equipment are many. Whatever contacts the living body, for example, the medical spatulas for depressing tongues or injection needles, deserve to be made into disposable products. However, it is not easy to leave a visible trace to an endoscope, even they have just been used and concern about unscrupulous people reusing the endoscopes exists.

US 8,562,514 B2 discloses a generically control method for a gastrointestinal tract diagnostic device with a disposable endoscope and a generically gastrointestinal tract diagnostic device with a disposable endoscope.

US 2005/0159646 A1 discloses a discloses an optical probe accessory device for use in vivo diagnostic procedures. The accessory device acts as an intermediate between the optical probe and the target tissue being analyzed. After use the accessory device is discarded e.g. by breakable elements to allow physical breakage of at least a portion of the accessory device upon removal from the probe or by an electronic element to use the endoscope again.

### SUMMARY OF THE INVENTION

The object of the instant disclosure is to provide a gastrointestinal tract diagnosis device with disposable endoscope and control method for the same to address the concern of the endoscope being repeatedly used and to improve the public health issue of unusual infection and spreading of pathogen. The object is solved by a control method according to claim 1 and a gastrointestinal tract diagnosis device according to claim 5.

To address the aforementioned deficiencies and inadequacies, the instant disclosure provides a control method for a gastrointestinal tract diagnosis device with disposable endoscope, at least comprising the following steps: (a) providing an endoscope, and electrically connecting the endoscope with a work station; (b) determining a usage state of the endoscope; and (c) determining the endoscope as being unused, being in use, or being using finished according to the usage state of the endoscope, wherein as the usage state of the endoscope is determined as being using finished, prohibit the usage of the endoscope.

To address the aforementioned deficiencies and inadequacies, the instant disclosure also provides a gastrointestinal tract diagnosis device with disposable endoscope, at least comprising: an endoscope having a circuit module, the circuit module at least including a substrate and a control unit and a recognition marker that are disposed on the substrate; an image capturing module at least including a first lens and an image sensor that are disposed on the substrate; an illuminating module at least including a first light source disposed on the substrate; an enclosure body assembled to a connection device to waterproofingly enclose the circuit module, the image capturing module and the illuminating module; and a work station connecting to the endoscope, so as to access the recognition marker and to add a machine identity into the recognition marker to match with the endoscope, wherein the work station determines if a usage state of the endoscope is overdue by a timer; and the work station determines if the endoscope has connected to an another work station different to the work station by the recognition marker and the machine identity.

In summary, a method for forcibly disabling the function of the endoscope is provided in the instant disclosure, allowing the endoscope to become a disposable endoscope only for one-time checking. The threats of unusual infection and spreading of pathogen can be prevented and the problems of public health can be solved.

Advantages and the essence of the instant disclosure can be further understood by the following detailed description provided along with illustrations to facilitate the disclosure of the present invention without limiting the same.

### BRIEF DESCRIPTION OF THE DRAWINGS

Fig. 1 is a schematic diagram of function blocks illustrating the gastrointestinal tract diagnosis device of the first embodiment of the instant disclosure.
Fig. 2A is an exploded schematic diagram from a perspective view illustrating the endoscope of the first embodiment of the instant disclosure.
Fig. 2B is an exploded schematic diagram from another perspective view illustrating the endoscope of the first embodiment of the instant disclosure.
Fig. 2C is a schematic diagram from a perspective view illustrating the endoscope without the enclosure body of the first embodiment of the instant disclosure.
Fig. 2D is a schematic diagram from a cross-section view illustrating the endoscope of the first embodiment of the instant disclosure.
Fig. 2E is a schematic diagram from a perspective view illustrating the endoscope of the first embodiment of the instant disclosure.
Fig. 3A is a schematic diagram from a top view illustrating the magnetic control device of the first embodiment of the instant disclosure.
Fig. 3B is a schematic diagram form a perspective view illustrating the magnetic control device of the first embodiment of the instant disclosure.
Fig. 4A is a schematic diagram illustrating a state of being used for viewing a cavity body by the instant disclosure of the first embodiment.
Fig. 4B is another schematic diagram illustrating a state of being used for viewing a cavity body by the instant disclosure of the first embodiment.
Fig. 4C is a schematic diagram illustrating a usage state of the instant disclosure being used for viewing a stomach.
Fig. 5 is a schematic diagram of a process illustrating the control method of the gastrointestinal tract diagnosis device of the first embodiment of the instant disclosure.
Fig. 6 is a schematic diagram of a process illustrating a control method of the instant disclosure.
Fig. 7 is a schematic diagram of function blocks illustrating the instant disclosure.

### DETAILED DESCRIPTION OF THE PREFERRED EMBODIMENTS

### [First embodiment]

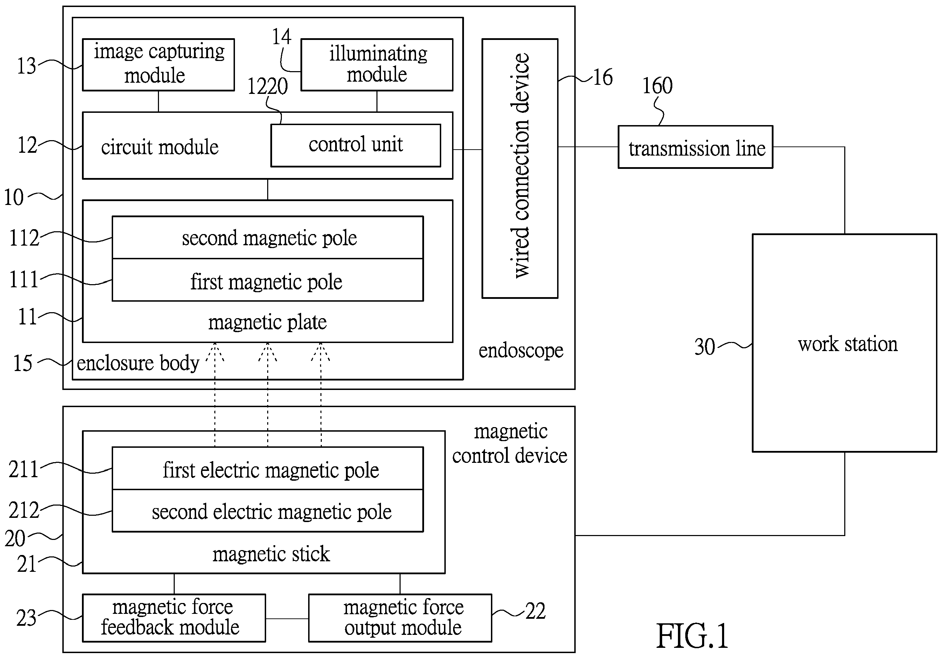

Please refer to Fig. 1, the instant disclosure provide a gastrointestinal tract diagnosis device, at least including: an endoscope 10 and a magnetic control device 20. The endoscope 10 and the magnetic control device 20 can be connected to a work station 30.

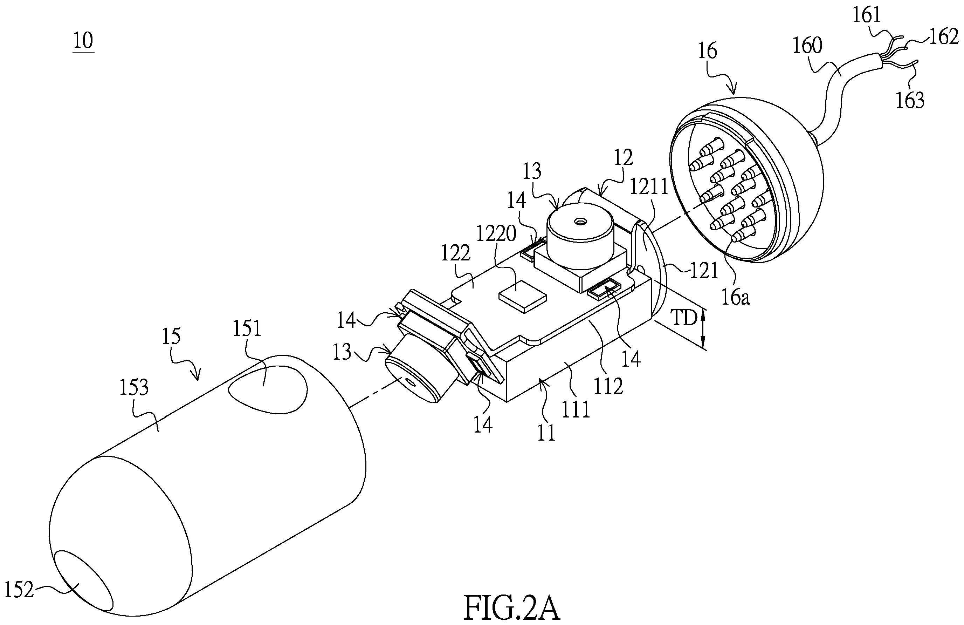

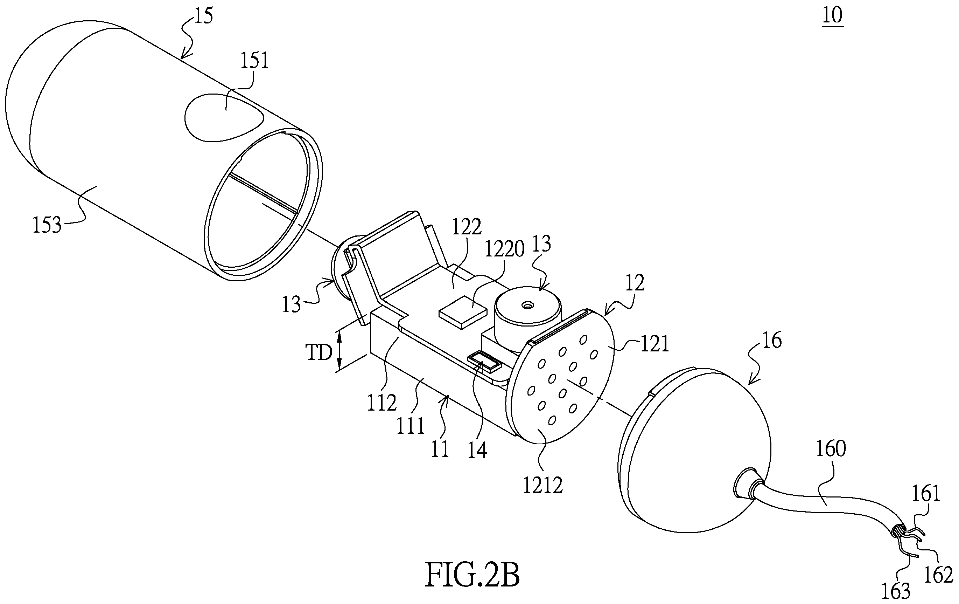

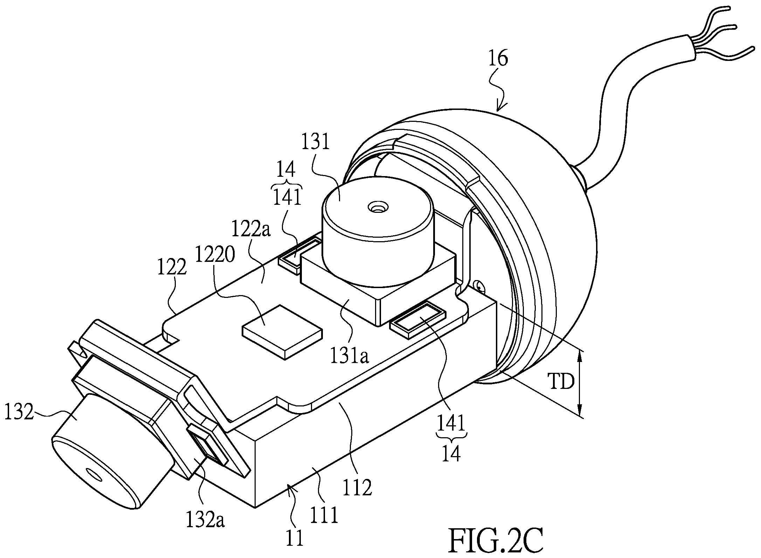

Please refer to Fig. 1, Fig. 2A, Fig. 2B and Fig. 2C, the endoscope 10 has a magnetic plate 11 in flat-plate form therein. A first magnetic pole 111 and a second magnetic pole 112 of the magnetic plate 11 respectively distribute at a lower half portion (label not shown) and an upper half portion (label not shown) of the magnetic plate 11 located along a thickness direction TD of the magnetic plate 11. The magnetic plate 11 can be in the form of a rectangular body, and can be a permanent magnet. Magnetic polarities of the first magnetic pole 111 and the second magnetic pole 112 are opposite to each other. As long as the polarities of the second magnetic pole 112 and the first magnetic pole 111 are opposite to each other, the first magnetic pole 111 is not limited to be N polar only or S polar only, and neither is the second magnetic pole 112 limited.

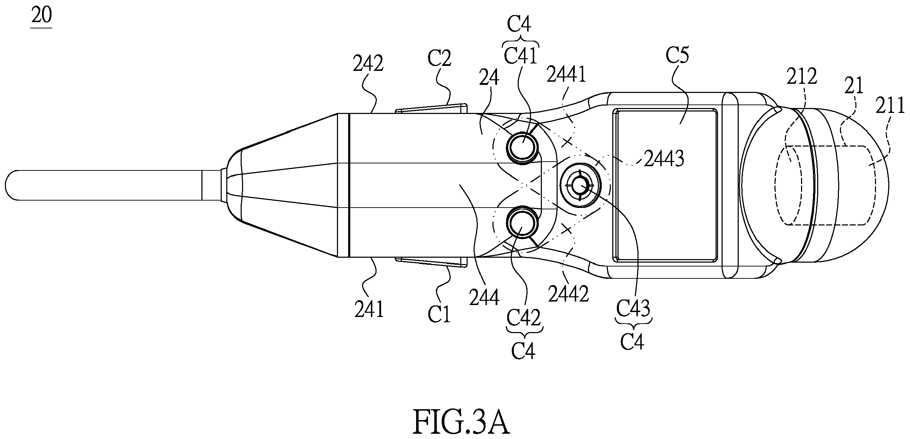

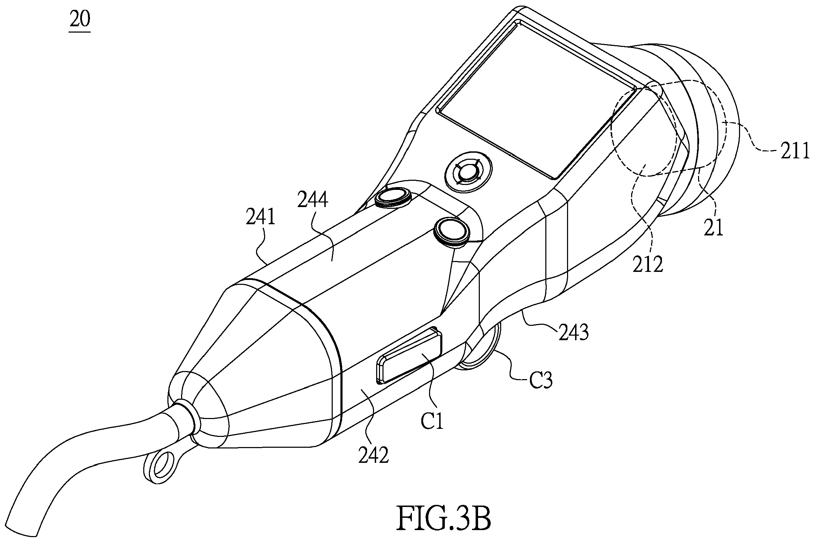

Please refer to Fig. 1, Fig. 3A and Fig. 3B, the magnetic control device 20 includes a magnetic stick 21. At least one electromagnet (not shown) made by an electromagnetic coil (not shown) can be disposed within the magnetic stick 21 so that a first electric magnetic pole 211 is formed at a terminal portion (label not shown) of the magnetic stick 22, and a second electric magnetic pole 212 is formed at a base portion (label not shown) of the magnetic stick 21. In other words, the first electric magnetic pole 211 and the second electric magnetic pole 212 that are formed can be respectively located at two terminals of the electromagnetic coil. The first electric magnetic pole 211 is not limited to be N polar only or to be S polar only. As long as the polarity of the first electric magnetic pole 211 is opposite to that of the second electric magnetic pole 212, the polarity of the second electric magnetic pole 212 is also not limited to be N polar only or to be S polar only. The first electric magnetic pole 211 can be used to selectively correspond to, as well as to attract (or to draw) or to repel, one of the first magnetic pole 111 and second magnetic pole 112 of the magnetic plate 11.

Please refer to Fig. 1 illustrating the schematic diagram of function blocks of the instant disclosure. For example, as first magnetic pole 111 of the magnetic plate 11 is set to be S polar, the first electric magnetic pole 211 can be N polar. The magnetic line of force, also equivalent to magnetic field and magnetic force, as schematically represented in form of the dotted line on Fig. 1, is oriented from a direction from the first electric magnetic pole 211 to the first magnetic pole 111 so that the magnetic stick 21 is able to control the movement and rotation of the endoscope 10 by the attraction of the magnetic force. In order to make the first electric magnetic pole 211 and the second electric magnetic pole 212 of the magnetic stick 21 generate magnetic force, the magnetic control device 20 further includes a magnetic force output module 22 for outputting a predetermined current to supply to the magnetic stick 21. The predetermined current supplied is able to be output and form a predetermined magnetic force on the first electric magnetic pole 211 and the second electric magnetic pole 212 according to the principle of electromagnetism. In other words, the magnetic force output module 22 has the magnetic stick 21 generate the predetermined magnetic force for acting on the first magnetic pole 111 and/or the second magnetic pole 112 of the magnetic plate 11 by the way of outputting the predetermined current to the magnetic stick 21. The predetermined magnetic force is applied as a force at a distance to attract or repel the magnetic plate 11. Taking this embodiment for instance, the first electric magnetic pole 211 becomes N polar from which the predetermined magnetic force is generated to be further applied for controlling the movement of the endoscope 10 by attracting the first magnetic pole 111, exhibiting polarity S, of the magnetic plate 11.

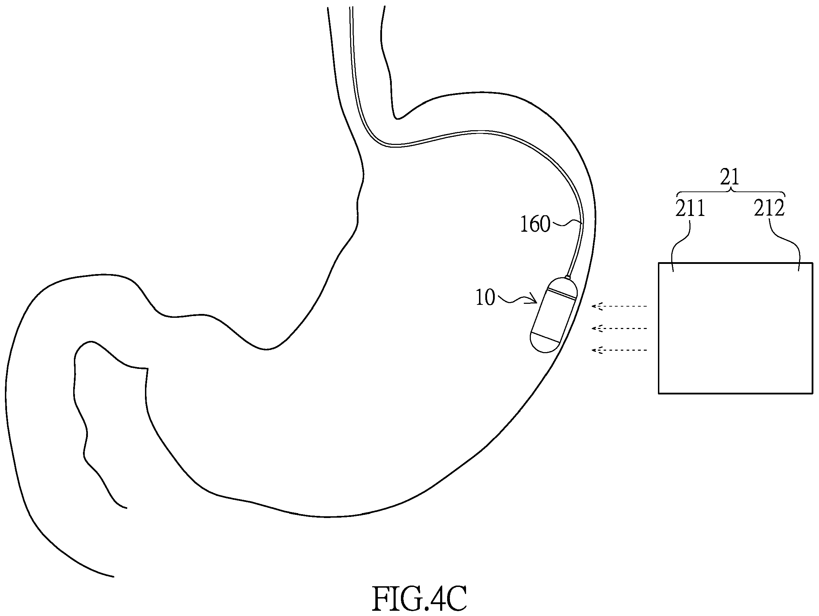

Preferably, the magnetic control device 20 further includes a magnetic force feedback module 23. Because the magnetic plate 11 can also cause a feedback of magnetic force to a sensor (not shown) within the magnetic stick 21 and change the predetermined current into an offset current, resulting in a variation of the predetermined magnetic force, subsequently, the magnetic force, for attracting the magnetic plate 11 within the endoscope 10, given off from the magnetic control device 20 becomes unstable, leading the endoscope 10 to be in an unstably-swaying condition. Moreover, as the magnetic control device 20 is in the process of drawing the endoscope 10, the magnetic control device 20 and the endoscope 10 get closer and closer to each other, resulting in stronger and stronger attraction force leading to an even closer distance between each other, making it easy for the endoscope 10 to abut the inner wall of the gastrointestinal tract such as the gastric wall (Fig. 4C, label not shown) with over strength. However, with the aid of the magnetic force feedback module 23, the return of the default predetermined current from the offset current so as to stabilize the output of the predetermined magnetic force can be achieved. Hence, the magnetic force feedback module 23 is helpful for stabilizing the control of the endoscope 10 by the magnetic control device 20. The sway of the endoscope 10 can be decreased. The situation that the gastric wall being hit by over strength due to the excessive magnetic attraction force can be also improved, helping decrease possible discomfort or other potential physical injury that may be brought on to the check recipients during the process of checking.

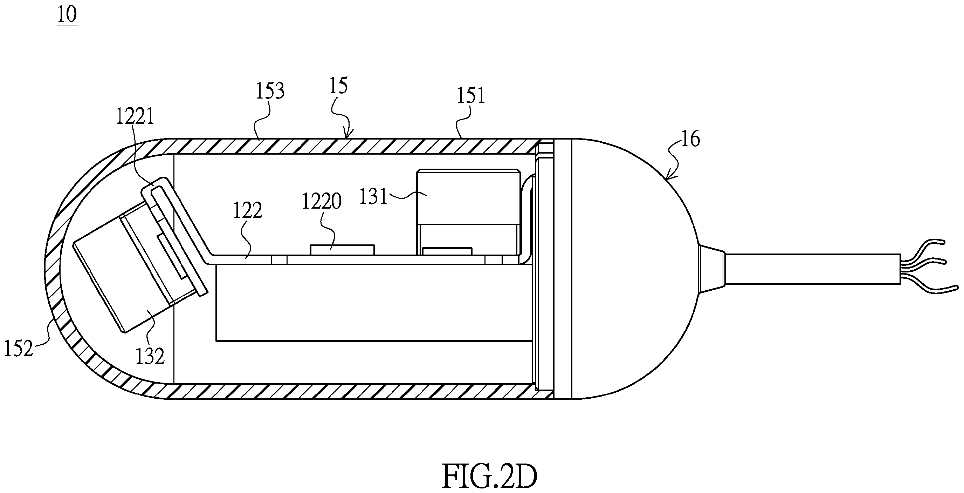

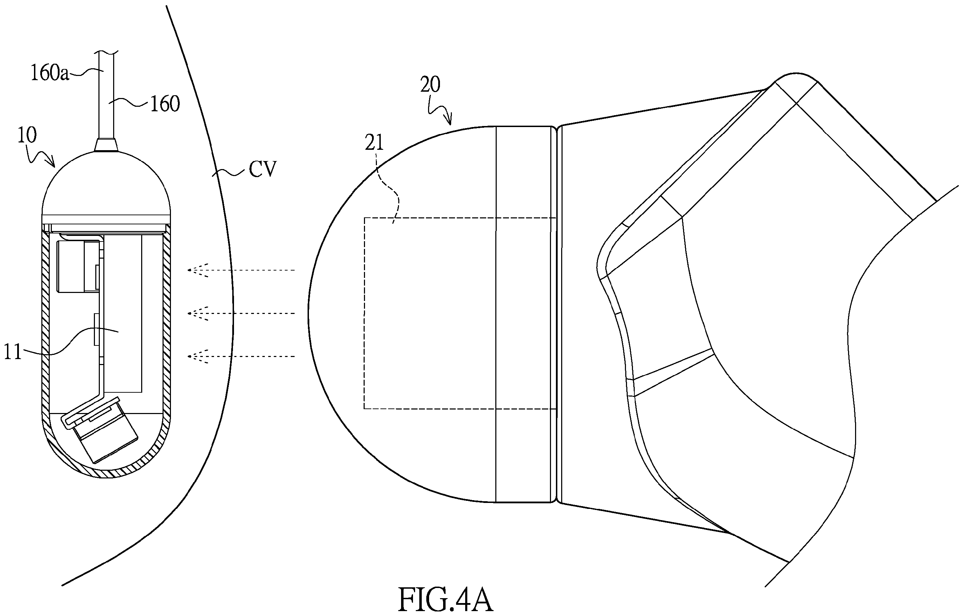

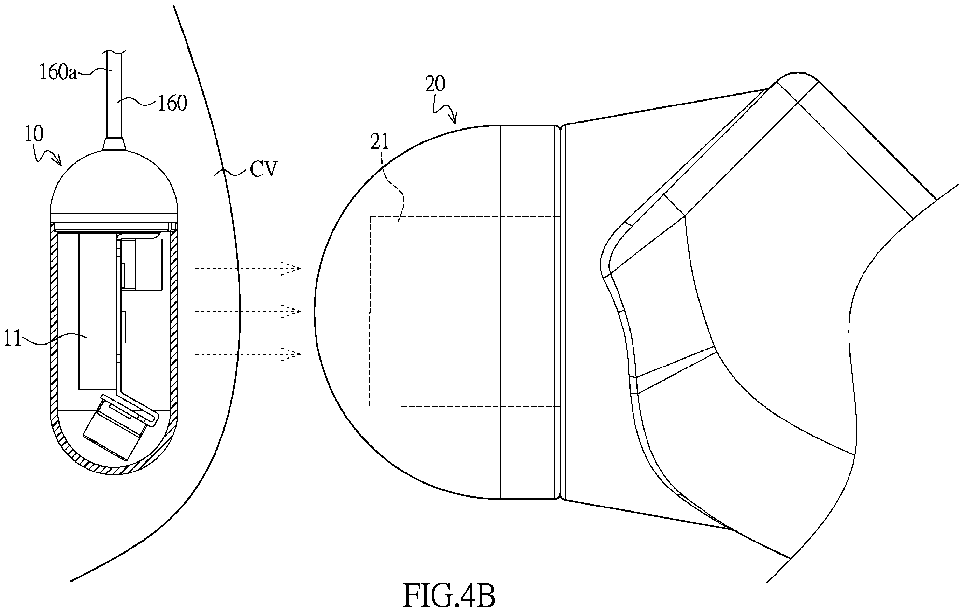

Please refer to Fig. 1, 2A and 2B, the endoscope 10 also includes a circuit module 12, an image capturing module 13, an illuminating module 14, an enclosure body 15 and a wired connection device 16. The circuit module 12 at least includes a conductive base 121, a substrate 122 extending from the conductive base 121 and a control unit 1220 being disposed on the substrate 122. The conductive base 121 is electrically assembled to a conductive interface 16a of the wired connection device 16. Preferably, the circuit module 12 can be made of a flexible circuit board. In other words, the substrate 122 can be in the form of a flexible circuit board. Please refer to Fig. 2C and Fig. 2D, the image capturing module 13 at least includes a first lens 131 and an image sensor 131a disposed on the substrate 122. The image sensor 131a would be a CCD (charge-couple device) or a CMOS (complementary metal-oxide-semiconductor) for image sensing. The illuminating module 14 includes a first light source 141. The first light source 141 can be disposed on a location adjacent to the first lens 131, providing enough light when the first lens 131 is photo shooting. Preferably, the first lens 131 and the image sensor 131a could be disposed on a surface 122a of the substrate 122 to make it a substantial lateral and horizontal view so that image checking and recording of a lateral 360 degree panorama is able to be done as the endoscope 10 is hung and spins based on the transmission line 160 (referring to the magnetic control device 20 illustrated in Fig. 4A and 4B).

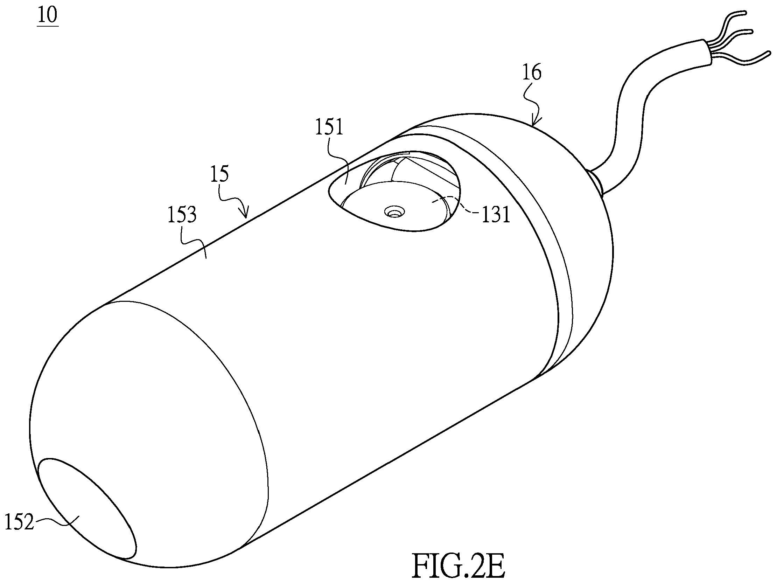

Referring to Fig. 1, Fig.2A, Fig. 2B and Fig. 2E, the enclosure body 15 is assembled with the wired connection device 16 along a direction starting from a first side 1211 of the conductive base 121 toward the wired connection device 16 so that the enclosure body 15 can enclose the circuit module 12 as well as the image capturing module 13, the illuminating module 14 and other related elements or modules having been disposed on the circuit module 12. As the enclosure body 15 is assembled with the wired connection device 16, a water proofing means can be accomplished and provided, rendering the instant disclosure to be a kind of capsule-type endoscope to protect the elements relating to the endoscope 10 within the enclosure body 15 from being destroyed by penetrated liquid. In addition, the wired connection device 16 is assembled with the enclosure body 15 along a direction defined from a second side 1212 of the conductive base 121 to have the ability of being waterproof. The wired connection device 16 electrically connects to the control unit 1220 on the circuit board 12 through the conductive base 121 and the electrical connection is further extended to other elements or modules from the control unit 1220. Preferably, the effect of being waterproof can be achieved by pouring some waterproof gel within any joint (not shown) that may be formed between the wired connection device 16 and the enclosure body 15, but is not limited thereto. A transmission line 160 is extended from the wired connection device 16. The transmission line 160 at least includes a signal transmission line 161 and a power transmission line 162 responsible for transmission of an audio or video signal and providing power, respectively. The endoscope 10 can also equipped with a microphone (not shown) therein to connect with the signal transmission line for audio. Preferably, the transmission line 160 can also include a grounding line 163. Each of the signal transmission line 161, power transmission line 162, and grounding line 163 has a terminal for electrically connecting to the circuit module 12 through the conductive base 121 by the conductive interface 16a. Each of the signal transmission line 161, power transmission line 162 and grounding line 163 has the other terminal being extended therefrom to electrically connect to a work station 30 (shown on Fig. 1) outside of the endoscope 10. The work station 30 can be a PC equipped with a monitor and a receiver (not shown), or the receiver can be a transceiver for receiving signals transmitted from the transmission line 160 and the monitor is for displaying the received signal of the video and the result. The monitor shows the real-time or non-real-time images the endoscope 10 captured. The work station 30 also records and saves the images received from the receiver/transceiver, being able to function as document management. The magnetic plate 11 can be fixed onto the circuit module 12, especially onto one side of the circuit module 12 and parallel to the circuit module 12. Specifically, one magnetic pole of the magnetic plate 11 is adhered to the lower surface of the substrate 122, but is not limited thereto.

Please refer to Fig. 2C. Preferably, the image capturing module 13 can also include a second lens 132 to form a duo lenses module. The second lens 132 is also equipped with an image sensor 132a. As Fig. 2D illustrates, a holder 1221 is slantingly extended from a free end (label not shown), distal to the wired connection device 16, of the substrate 122. The second lens 132 is disposed on the holder 1221, allowing the direction of the second lens 132 to form an angle with a lengthwise axis of the endoscope 10. When the endoscope 10 is controlled by the magnetic control device 20 (as shown in Fig. 4A and 4B) to axially rotate based on the lengthwise axis, the second lens 132 is disposed on the slantingly oriented holder 1221 so that a bigger view can be broadened by the second lens 132. To provide enough light to the second lens 132, a second light source (label not shown) can also be disposed on the holder 1221. Preferably, a light-emitting diode (LED) can be used for the second light source and the first light source 141.

Please refer to Fig. 2D and Fig. 2E. Preferably, the enclosure body 15 includes a first polished region 151, a second polished region 152 and a matte uniformity region 153. The first polished region 151 corresponds to the first lens 131. The first polished region 151 is formed by a polishing on a part of the surface of the enclosure body 15 so that the partial surface of the enclosure body 15 becomes smooth, allowing light to pass through the enclosure body 15 without any blocking, to enter the first lens 131 and allowing the image to be captured by the lens clearly. The second polished region 152 is corresponding to the second lens 132. Excepting for the first polished region 151 and the second polished region 152, the rest of the regions of the enclosure body 15 can be atomized, for example, but not limited thereto, to treat the surface of the enclosure body 15 by sandblasting so that a matte uniformity region 153 is formed. In such a way, any improper light reflection from the rest of the parts of the surface of the enclosure body 15, except for the first polished region 151 and the second polished region 152, that causes interference to the image capturing can be avoided.

Referring to Fig. 1, 3A and 3B, the magnetic device 20 further includes a holding portion 24 connected with the base portion of the magnetic stick 21. The holding portion 24 includes a right side 241, a left side 242, a belly portion 243 and a back portion 244. The right side 241 has a first booting button C1 used for being pressed. The left side 242 has a second booting button C2 used for being pressed. The belly portion 243 has a ring C3 thereon. The ring C3 is preset in an off-stated default. The back portion 244 has manipulation buttons C4 and a screen C5 thereon. The manipulation buttons C4 are pre-configured in an off-stated default. Both the first booting button C1 and the second booting button C2 can be prepressed to unlock the off-stated default of the ring C3 and the manipulation buttons C4. In detail, users have to prepress either the first booting button C1 or the second booting button C2 so as to active the control function of the ring C3 and the manipulation buttons C4. The belly portion 243 and the magnetic stick 21 form an angle between 155 degree and 175 degree, making it fairly similar to say that the holding portion 24 and the magnetic stick 21 form an angle between 155 degree and 175 degree to form a gun-like structure. It is convenient for users to hold and control direction of the magnetic control device 20. Take the gun-like structure for example, when right-handed users hold the holding portion 24, the first booting button C1 will be firstly and simultaneously pushed by the palm of the right hand, but the second booting button C2 will not be pressed. Hence, the off-stated default of the ring C3, manipulation buttons C4 and screen C5 would be unlocked to recover the control function. The forefinger of the right hand can easily hook to the ring C3 and control the rotation of the first electric magnetic pole 211 and the second electric magnetic pole 212 of the magnetic stick 21 by triggering the ring C3 forward or backward so as to drive the endoscope 10 to rotate or move. For instance, the magnetic stick 21 can be driven to rotate clockwise by pushing the ring C3 forward, or can be driven to rotate counter clockwise by pulling the ring C3 backward, helping the endoscope 10 be positioned in a specific direction to obtain the desired sight for shooting or observing. As Fig. 3A shows, the thumb of the right hand could access to the manipulation buttons C4 to press the left key C41 of the manipulation buttons C4 and press the menu key C43 of the manipulation buttons C4 without resulting in unstable holding. In other words, the left key C41 can be arranged on the first location 2441 of the back portion 244, and the first location 2441 can be pressed by the thumb of the right hand. Likewise, the second booting button C2 on the left side 242 and the right key C42 on the manipulation buttons C4 is suitable for left-handed users. The manipulation circumstance for left-handed users is analog to that for the right-handed users, except the different orientations of use resulting from right hand and the left hand and unnecessary details for left handed users will not be introduced again here. The right key C42 can be arranged on a second location 2442 on the back portion 244. The second location 2442 can be pressed by the thumb of the left hand. Preferably, the menu key C43 can be arranged on a third location 2443 on the back portion 244. The third location 2442 is a location that can be pressed by the thumbs of the right hand and the left hand. The third location would be a place defined by the overlap of the first location 2441 and the second location 2442. Referring to Fig. 1 and 3A, the left key C41 and the right key C42 are mainly used for controlling and initiating the shooting of the image capturing module 13 therefore the left key C41 and the right key C42 can further be a first two-stage button and a second two-stage button, respectively. They are similar to shutter keys of a camera when being clicked down to the first stage, focusing of the endoscope 10 is carried out, and when being clicked down to the second stage, image shooting of the endoscope 10 is performed. For the convenience of saving images, a memory card reader (not shown) can be further arranged on the holding portion 24 to access the image file anytime, however work station 30 can be also responsible for saving and recording work.

Please refer to Fig. 1, 4A, 4B and 5, according to the above technical contents, the instant disclosure further provides a control method of a gastrointestinal tract diagnosis device, at least including the following steps.

Step S101: providing an endoscope 10 having a transmission line 160 and a magnetic plate 11, and delivering the endoscope 10 into a cavity body CV with a first section 160a of the transmission line 160 and a second section (not shown) of the transmission line 160 being left outside of the cavity body CV and being connected to a work station 30. The cavity body CV could be a cavity of a living body or a bag-shaped cavity with two openings respectively being opened at two terminals on the cavity and connecting to the cavity, for example, the stomach, but is not limited thereto, therefore the cavity could be one of a non-living body. Taking the stomach for demonstration, preferably, the endoscope 10 with the first section 160a of the transmission line 160 can be delivered into the stomach by swallowing. Thus, the transmission line 160 would be a soft line made of insulation material having high biocompatibility. The transmission line 160 is different from the hard tube of known endoscope and it would be better for the transmission line 160 to have a smaller diameter to avoid discomfort caused on check recipients. Preferably, the transmission line 160 could have a diameter ranging from 1.4 to 2.4 centimeter (cm), and this diameter range basically approaches to the diameter of a common adult. Nevertheless, the above diameters are merely for being references and are not limited thereto. In particular, as long as the diameter of the transmission line 160 conforms to that of a person's esophagus and the esophagus feeling of being invaded by foreign material caused to a swallower is largely decreased, the bottom limit of the diameter is not limited and diameter of the transmission line 160 should be viewed at its best size. It is believed that the feeling of fear and being invaded could be decreased by swallowing the endoscope 10 with transmission line 160 at the best diameter. Besides, it also helps to allow a section of the transmission line 160 to get in the cavity body CV along with the endoscope 10 and the other section (not shown) of the transmission 160 would be left outside of the cavity body CV. The retaking of the instant disclosure after being used would not be a problem.

Step S103 includes: providing a magnetic control device 20 at least having a magnetic stick 21 and a magnetic force output module 22, and activating the magnetic control device 20 to allow the magnetic force output module 22 to generate a predetermined magnetic force so that one of the two electric magnetic poles could be used to electro-magnetically attract the magnetic plate 11 within the endoscope 10. Then the observation of the cavity body CV could be done by attracting / drawing the endoscope 10 toward an appropriate view by the magnetic control device 20.

Step S105 includes: adjusting the movement, rotation and view field of the endoscope 10 inside the cavity body CV by means of the magnetic control device 20 to record images of the inner wall of the cavity body CV.

Step S107 includes: repeating step (b) to step (c) till the endoscope 20 finishes recording images of the inner wall of the cavity body CV. It is worth noting that during the process S107, the endoscope 10 carries out the recording basically after being drawn to an appropriate location inside the cavity body CV corresponding to the nidus and unnecessary redundant shooting as well as image recording would not result. The trouble of screening the huge amount of image data to know if any images have been recorded of any desired image of a nidus would be avoided.

Step S109 includes: terminating the magnetic control device 20 to cease the predetermined magnetic force.

Step S111 includes: pulling the second section (not shown) of the transmission line 160 to render the endoscope 10 moving from inside of the cavity body CV to outside of the cavity body CV, so as to retake the endoscope 10. Because the instant disclosure has the transmission line 160, the problem of being difficult to get the endoscope 10 out of the cavity body CV can be overcome.

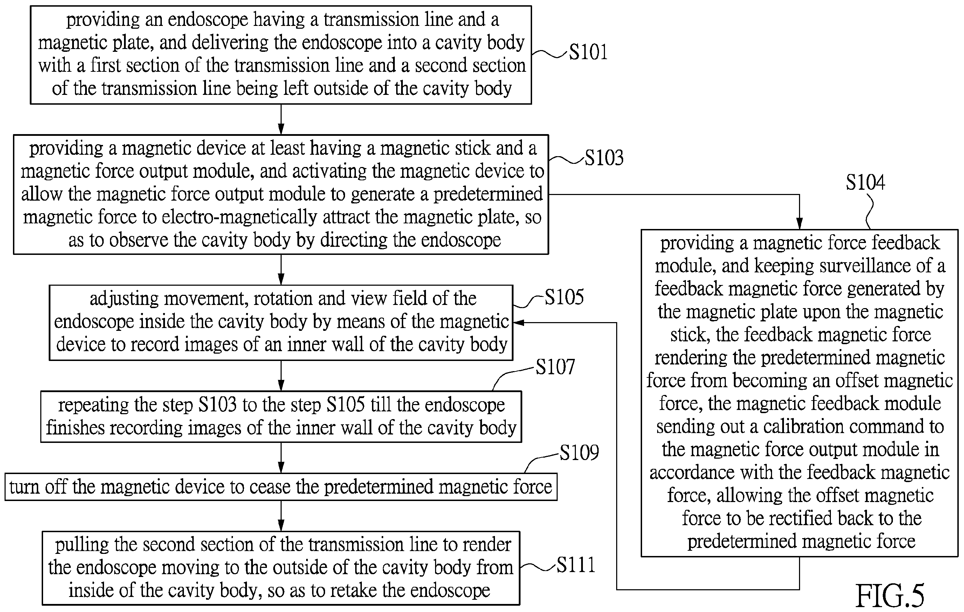

Preferably, as shown in Fig. 1 and 5, when the step S103 is progressed to the step S105, the following step S104 is further included: providing a magnetic force feedback module 23, and keeping surveillance of a feedback magnetic force generated by the magnetic plate 11 upon the magnetic stick 21, the feedback magnetic force rendering the predetermined magnetic force from becoming an offset magnetic force, by the magnetic force feedback module 23 sending out a calibration command to the magnetic force output module 22 in accordance with the feedback magnetic force, allowing the offset magnetic force to be rectified back to the predetermined magnetic force. As the step S103 to the step S105 is repeated within the above step S107, the step S104 can also be added between step S103 and S105 for being repeated. In addition, during progression of step S103 to step S105, real-time image recording and displaying according to the view of the endoscope 10 can be done by means of the work station 30 being connected with the transmission line 160. It is understood that the instant disclosure has the advantages of shooting and displaying real-time images.

### [second embodiment]

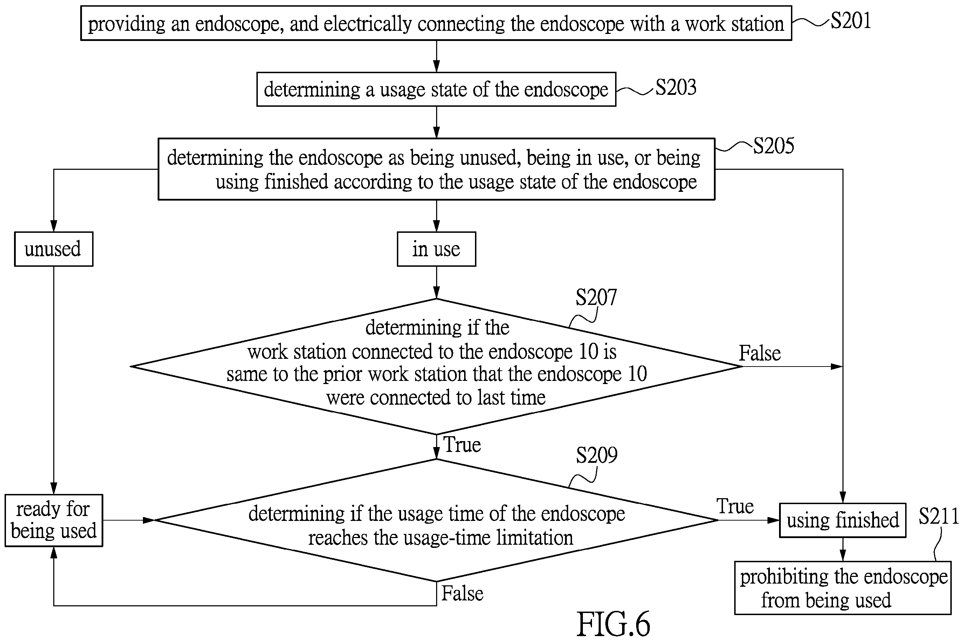

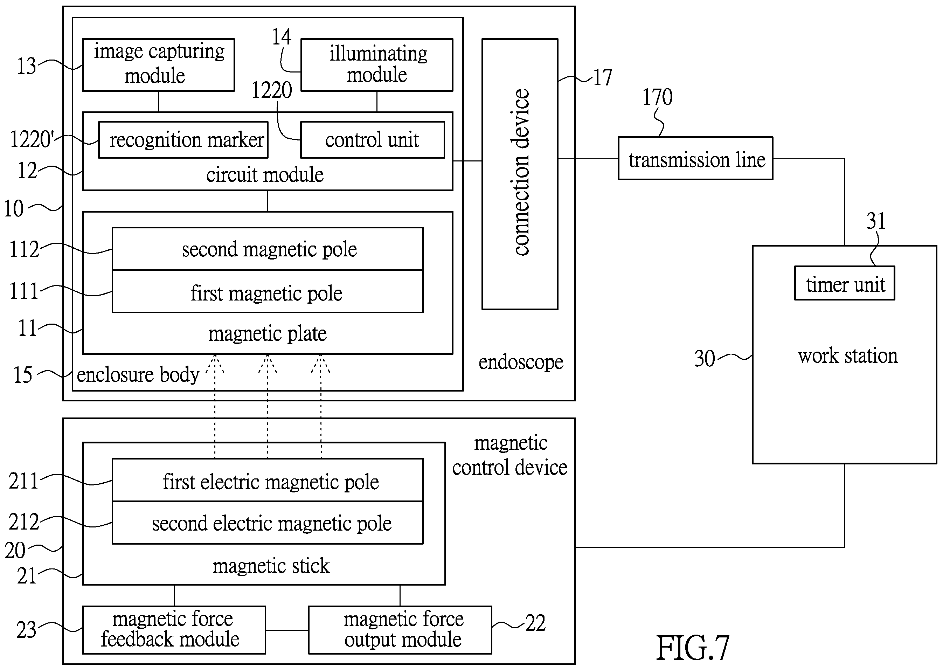

Referring to Fig. 6 and 7, the instant disclosure further provides a control method for a gastrointestinal tract diagnosis device with disposable endoscope. The control method at least includes the following steps: step S201: providing an endoscope 10, and connecting the endoscope 10 with a work station 30. Step S203: determining a usage state of the endoscope 10. Step S205: determining the endoscope 10 as being not yet used, being in use, or being out of use according to the usage state of the endoscope 10. As the usage state of the endoscope 10 is determined as being out of use, the step S211 is directly carried out to prohibit the usage of the endoscope.

If the usage state of the endoscope 10 is determined as being not yet used, go on to carry out the step S209 to determine if a usage time of the endoscope 10 reaches a usage-time limitation till the usage time reaches the usage-time limitation, then determining the usage state of the endoscope 10 as being out of use, subsequently carrying out the step S211 to prohibit the usage of the endoscope 10.

If the usage state of the endoscope 10 is determined as being in use, go on to determine if the work station 30 that the endoscope is connected to is the same as a prior work station (not shown) that the endoscope 10 was connected to last time. If the work station 30 is different from the prior work station having been connected to last time, determine the endoscope 10 as being out of use and carry out the step S211 to prohibit the endoscope 10 from being used. On the other hand, if the work station 30 is confirmed to be the same as the prior work station 30 that the endoscope 10 was connected to last time by carrying out step S207, go on to carry out the step S209 to determine if the usage time of the endoscope 10 reaches the usage-time limitation. If the result of the step S209 is "False", the endoscope 10 can be continued to be operated and the step S209 is returned to make determination of the endoscope 10 till the usage time of the endoscope 10 reaches the usage-time limitation which means the result of the step S209 is "True" so that the usage state of the endoscope 10 is determined as being out of use and the step S211 is carried out to prohibit the endoscope 10 from being used.

Preferably, the step S207, to determine if the work station 30 being connected to by the endoscope 10 is the same as the prior work station that the endoscope 10 was connected to last time, is based on the checking of the machine identity (e.g. serial number of firmware) of the work station 30 and the machine identity of the prior work station. In detail, the prior work station 30 that was connected to can be matched based on a recognition marker 1220' of the endoscope 10, and the recognition marker 1220' can be added with the machine identity of the prior work station by means of the prior work station. Thus, the work station 30 can determine if the machine identity of the prior work station is the same as that of the work station 30 present to determine if the work station 30 the endoscope 10 being connected to the same as the prior work station. The above disclosure is a preferable demonstration but is not limited thereto.

Preferably, the endoscope 10 can be preset to have a period of usage-time limitation. In other words, the usage of the endoscope 10 can be restricted by the usage-time limitation. The usage-time limitation can be delimited to at least include an initial value and a final value. The aforementioned step S201 further include the steps of initiating a timer unit 31 of the work station 30 to generate a timing value according to the timer unit 31. The timer unit 31 can run the work of timing in a way of counting up or counting down. Despite counting up or counting down, an initial value and a final value can be generated. Thus, the way that the timer unit 31 works in the instant embodiment is not limited to ways of counting up or counting down. If the timing value is equal to the initial value, the endoscope 10 is determined as being unused. If the timing value is equal to or beyond the final value, the usage state of the endoscope 10 is determined as being using finished in step S205. If the timing value is between the initial value and the final value, the usage state of the endoscope 10 is determined as being in use. Preferably, the aforementioned usage-time limitation can be preset depending to demands, e. g. usage-time limitation of 10 to 30 minutes, as products are being finalized and leaving the factory but is not limited thereto. However, if it is to be used for viewing the gastrointestinal tract, generally speaking, 10 minutes of usage-time limitation would be enough. Furthermore, one of the ways to prohibit the endoscope 10 from being used by means of the work station 30 as well as software installed therein is to have the endoscope 10 disabled, to have the endoscope 10 unable to be activated, or to have the fuse (not shown) in the endoscope 10 cut by inputting an instantaneous electrical power of low voltage and high current, so as to prohibit the endoscope 10 from being used. The aforementioned fuse can be installed in the circuit of the circuit module 12 but is not limited thereto. The elements inside the endoscope 10 of this embodiment, e.g. the circuit module, can also be waterproofingly enclosed by the enclosure body 15 as introduced in the first embodiment. Because the aforementioned control method is realized through the satisfying of some requirements to forcibly prohibit the endoscope 10 from being used, the endoscope 10 is also forcibly turned into an endoscope 10 that must be discarded and replaced, so as to meet the purpose of having a disposable endoscope, of the instant disclosure.

Please refer to Fig. 7, another variant derived according to the first embodiment and the aforementioned control method. The different technical features of the variant of the second embodiment and the first embodiment can be commonly applied to each other. The instant embodiment provides a gastrointestinal tract diagnosis device with disposable endoscope, at least including an endoscope 10, and a work station 30. The work station 30 is connected to the endoscope 10. The endoscope 10 has a circuit module 12, an image capturing module 13, an illuminating module 14 and an enclosure body 15.

What is different from the first embodiment is that the circuit module 12 of the second embodiment at least includes a substrate 122, and a control unit 1220 as well as a recognition marker 1220' that are disposed in the substrate 122. The work station 30 can be used for connecting the endoscope 10 to read/access or write/add the machine identity of the work station 30 from or in the recognition marker 1220', so as to match with the endoscope 10. The recognition marker 1220' can be firmware for the purpose of recognition that is burned on the substrate 122 to hold information for recognition, such as a serial number or symbol, related to identifying the endoscope 10. In other words, the recognition marker 1220' not only can carry recognition information related to the endoscope 10 but also can be added or written in with information about the machine identity of the work station 30, allowing the work station 30 to determine if the endoscope 10 has connected to a prior work station different to the present work station 30 according to the recognition marker 1220' and the machine identity inside the recognition marker 1220'.

Referring to Fig. 2C, the similar part of the instant embodiment and the first embodiment is that the image capturing module 13 at least includes a first lens 131 as well as an image sensor 131a that are disposed on the substrate 122. The illuminating module 14 at least includes a first light source 141 disposed on the substrate 122. The enclosure body 15 is the same to the one shown in Fig. 5A except that the enclosure body 15 of the instant embodiment is not deemed to have to connect with a wired connection device. The enclosure body 15 connects to the connection device 17 of which the function is for waterproofingly enclosing the circuit module 122, image capturing module 13 and the illuminating module 14. In other words, the connection device 17 is not limited to have to be equipped with a transmission line 160 as the wired connection device 16 shown in Fig. 2A is. The connection device 17 can only be assembled with the enclosure body 15, rendering the endoscope 10 to become a wireless endoscope capsule. Therefore, the endoscope 10 is able to undergo wireless connection with the work station 30 under wireless condition, however, it is not limited thereto. Hence, the connection device 17 can also connect to the work station 30 through a wired manner.

Preferably, the work station 30 also has a timer unit 31. The work station 30 is able to determine if the usage state of the endoscope 10, carrying the same recognition marker 1220' as the work station 30, reaches or is over the preset usage-time limitation. Once the usage state of the endoscope 10 reaches or is over the time limit, the endoscope 10 is disabled in a way such as the aforementioned control method. The work station 30 can also determine if the endoscope 10 had been connected to a prior work station different from the work station 30 at present according to the recognition marker 1220' and the information of machine identity written in the recognition marker 1220'. A recognition unit (not shown) in the work station 30 can be used to identify the recognition marker 1220' to achieve the determination but is not limited thereto.

In the instant embodiment, a magnetic plate 11 can also be disposed inside the endoscope 10 and collocate with a magnetic control device 20 and the magnetic stick 21, the magnetic force output module 22, as well as the magnetic force feedback module 23 to work in a way similar to the aforementioned embodiments disclosed. In other words, the device as being disclosed in the first embodiment can also include some elements of the device as being demonstrated in the instant embodiment, making the endoscope 10 function as a disposable element. However, the instant embodiment still can escape from the typical model of the first embodiment to become a gastrointestinal tract diagnosis device with disposable endoscope independent from the first embodiment. Please refer to Fig. 4C. Fig. 4C illustrates a usage state of the instant disclosure being used for viewing a stomach. The magnetic stick 21, which has a first electric magnetic pole 211 and a second electric magnetic pole 212, is illustrated in a form of a rectangle. The first electric magnetic pole 211 is closer to the stomach (label not shown) than the second electric magnetic pole 212 is in Fig. 4C. The first electric magnetic pole 211 attracts / draws the endoscope 10 inside the stomach in a way of distal force and the movement of the endoscope 10 in the stomach is under control.

To sum up all of the above embodiments, more stable control of the endoscope can be achieved. The inappropriate physical harm to the cavity being viewed resulting from the endoscope can be avoided. In addition the instant disclosure is easy to be taken back or recycled. Therefore as the instant disclosure is delivered into the living body, the problem of being difficult to take the endoscope out won't be caused. Thus, unnecessary surgical as well as medical source waste will not be caused. After being recovered, the function of the endoscope can be forcibly disabled to solve the problem of endoscopes being repetitively used in checking gastrointestinal tracts of non-single-specific cases and the problem that the subsequent sterilizing can't be guaranteed. The descriptions illustrated supra set forth simply the preferred embodiments of the present invention; however, the characteristics of the present invention are by no means restricted thereto. All changes, alterations, or modifications conveniently considered by those skilled in the art are deemed to be encompassed within the scope of the present invention delineated by the following claims.

## Claims

1. A control method for a gastrointestinal tract diagnosis device with disposable endoscope (10), at least comprising the following steps:
(a) providing an endoscope (10), and electrically connecting the endoscope (10) with a work station (30);
(b) determining a usage state of the endoscope (10); and
(c) determining the endoscope (10) as being unused, being in use, or being using finished according to the usage state of the endoscope (10), wherein
if the usage state of the endoscope (10) is determined as being using finished, prohibiting the usage of the endoscope (10);
**characterized in,**
**that** if the usage state of the endoscope (10) is determined as being in use, keep determining if the work station (30) that the endoscope (10) is connected thereto is the same as another work station (30) that the endoscope (10) was connected to last time, wherein if a machine ID of the work station (30) is different from a machine ID of the other work station (30) being connected to last time, prohibit the usage of the endoscope (10).

2. The control method for a gastrointestinal tract diagnosis device with disposable endoscope (10) according to claim 1, wherein if the usage state of the endoscope (10) is determined as being unused, keep determining if a usage time reaches a usage-time limitation till the usage time reaches the usage-time limitation; determining the usage state of the endoscope (10) as being using finished, subsequently prohibiting the usage of the endoscope (10).

3. The control method for a gastrointestinal tract diagnosis device with disposable endoscope (10) according to claim1, wherein if the work station (30) is the same as the other work station (30) being connected to last time, keep determining if a usage time of the endoscope (10) reaches a usage-time limitation till the usage time reaches the usage-time limitation; determining the usage state of the endoscope (10) as being using finished, subsequently prohibiting the usage of the endoscope (10).

4. The control method for a gastrointestinal tract diagnosis device with disposable endoscope (10) according to claim 1, wherein the endoscope (10) is set to be restricted by a usage-time limitation, the usage-time limitation having an initial value and a final value, the step (a) including initiating a timing unit of the work station (30) to generate a timing counting value,
wherein as the timing counting value is equal to the initial value, determine the usage state of the endoscope (10) as being unused;
as the timing counting value is equal to or beyond the final value, determine the usage state of the endoscope (10) as being using finished; as the timing counting value is between the initial value and the final value, determine the usage state of the endoscope (10) as being in use.

5. A gastrointestinal tract diagnosis device with disposable endoscope (10), at least comprising:
an endoscope (10) having a circuit module (12), the circuit module (12) at least including a substrate (122) and a control unit (1220) that is disposed on the substrate (122);
an image capturing module (13) at least including a first lens (131) and an image sensor (131a) that are disposed on the substrate (122);
an illuminating module (14) at least including a first light source (141) disposed on the substrate (122);
an enclosure body (15) assembled to a connection device (16, or 17) to waterproofingly enclose the circuit module (12), the image capturing module (13)and the illuminating module (14); and
a work station (30),
wherein the work station (30) determines if a usage state of the endoscope (10) is overdue by a timer (31);
**characterized in,**
**that** the circuit module (12) comprises a recognition marker (1220' ) that is disposed on the substrate (122); and
the work station (30) is adapted to be connected to the endoscope (10), so as to access the recognition marker (1220' ) and to add a machine identity into the recognition marker (1220' ) to match with the endoscope (10); and
the work station (30) is further adapted to determine if the endoscope (10) had connected to an another work station (30) different to the work station (30) by the recognition marker (1220' ) and the machine identity;
wherein the endoscope (10) includes a magnetic plate (11) therein, the magnetic plate (11) having a first magnetic pole (111) and a second magnetic pole (112) respectively distributed at a lower half portion and an upper half portion of the magnetic plate (11) located along a thickness direction (TD) of the magnetic plate (11), the magnetic plate (11) being fixed with the circuit module (12);
wherein the gastrointestinal tract diagnosis device with disposable endoscope (10) further includes
a magnetic control device (20) including:
a magnetic stick (21) including a first electric magnetic pole (211) at a terminal portion of the magnetic stick (21) and a second electric magnetic pole (212) located at a base portion of the magnetic stick (21);
a magnetic force output module (22) adapted to output a predetermined current to the magnetic stick (21) the magnetic stick (21) adapted to generate a predetermined magnetic force for interacting with the first magnetic pole (111) and the second magnetic pole (112); and
a magnetic force feedback module (23),
wherein the predetermined magnetic force is adapted to draw the magnetic plate (11) to control the movement of the endoscope (10);
wherein the magnetic plate (11) is adapted to cause a feedback magnetic force to a sensor of the magnetic stick (21), converting the predetermined current into an offset current, and wherein the magnetic force feedback module (23) is adapted to calibrate the offset current resuming the offset current back to the predetermined current, so as to stabilize the predetermined magnetic force.

6. The gastrointestinal tract diagnosis device with disposable endoscope (10) according to claim5, wherein the connection device (16, or 17) further includes a conductive interface (16a) and a transmission line (160), wherein one terminal of the transmission line (160) connects with the circuit module (12) through the conductive interface;
the other terminal of the transmission line (170) extends out from the connection device (17) to connect to the work station (30).

7. The gastrointestinal tract diagnosis device with disposable endoscope (10) according to claim5, wherein a holder (1221) is slantingly extended from a free end of the substrate (122), and the first lens (131) is disposed on the holder (1221).

8. The gastrointestinal tract diagnosis device with disposable endoscope (10) according to claim5, wherein the image capturing module (13)includes a second lens (132), the second lens (132) being disposed on a surface (122a) of the substrate (122).

9. The gastrointestinal tract diagnosis device with disposable endoscope (10) according to claim5, wherein the enclosure body (15) includes a first polished region (151), a second polished region (152) and a matte uniformity region (153) thereon, wherein the first polished region (151) is corresponded to the first lens (131) and the second polished region (152) (152) is corresponded to the second lens (132).

10. The gastrointestinal tract diagnosis device with disposable endoscope (10) according to claim 5, wherein the magnetic control device (20) includes:
a holding portion (24) connected with the base portion of the magnetic stick (21), the holding portion (24) including:
a first side (241) with a first booting button (C1) preset in an off-stated default thereon;
a second side (242) with a second booting button (C2) preset in an off-stated default thereon;
a belly portion (243) with a ring (C3) thereon for controlling a spin state of the magnetic stick (21); and
a back portion (244) with manipulation buttons (C4) and a screen (C5) thereon, wherein the first booting button (C1) and the second booting button (C2) are for being pressed firstly for respectively unlocking the off-stated default of the ring (C3) and the manipulation buttons (C4).

11. The gastrointestinal tract diagnosis device with disposable endoscope (10) according to claim 10, wherein the manipulation buttons (C4) includes buttons of menu (C43), a first key (C41) and a second key (C42),
wherein the first key (C41) is arranged on a first location (2441) of the back portion (244), so as to be capable of being pressed by a thumb of a right hand;
the second key (C42) is arranged on a second location (2442) of the back portion (244), so as to be capable of being pressed by a thumb of a left hand; and
the buttons of menu (C43) is arranged on a third location (2443) of the back portion (244), so as to be capable of being pressed by the thumb of either the left hand or the right hand.

12. The gastrointestinal tract diagnosis device with disposable endoscope (10) according to claim10, wherein the belly portion (243) and the magnetic stick (21) form an angle between 155 degree and 175 degree.

13. The gastrointestinal tract diagnosis device with disposable endoscope (10) according to claim11, wherein the belly portion (243) and the magnetic stick (21) form an angle between 155 degree and 175 degree.

14. The gastrointestinal tract diagnosis device with disposable endoscope (10) according to claim11, wherein the first key (C41) and the second key (C42) are a first key of two-stage pushing and a second key of two-stage pushing, respectively.

## Patentansprüche

1. Steuerungsverfahren für eine Gastrointestinaltraktdiagnosevorrichtung mit einem verfügbaren Endoskop (10), das zumindest die folgenden Schritte umfasst:
(a) Bereitstellen eines Endoskops (10) und elektrisches Verbinden des Endoskops (10) mit einer Arbeitsstation (30),
(b) Ermitteln eines Verwendungsszustands des Endoskops (10), und
(c) Ermitteln ob das Endoskop (10) nicht in Gebrauch befindlich, in Gebrauch oder der gebrauchsabgeschlossen ist, entsprechend dem Verwendungszustand des Endoskops (10), wobei
wenn der Verwendungszustand des Endoskops (10) als Gebrauch abgeschlossen ermittelt wird, wird die Verwendung des Endoskops (10) verboten,
**dadurch gekennzeichnet, dass**,
wenn der Verwendungszustand des Endoskops (10) als im Gebrauch ermittelt wird, weiter ermittelt wird, ob die Arbeitsstation (30), mit der das Endoskop (10) verbunden ist, dieselbe ist wie eine andere Arbeitsstation (30), mit der das Endoskop (10) das letzte mal verbunden war, wobei wenn eine Maschinen-ID der Arbeitsstation (30) von einer Maschinen-ID der anderen Arbeitsstation (30), die letztes Mal verbunden war unterschiedlich ist, die Verwendung des Endoskops (10) verboten wird.

2. Steuerungsverfahren für eine Gastrointestinaltraktdiagnosevorrichtung mit einem verfügbaren Endoskop (10) nach Anspruch 1, wobei wenn ermittelt wird, dass der Verwendungszustand des Endoskops (10) als nicht in Gebrauch befindlich ist, weiter ermittelt wird, ob eine Verwendungszeit eine Verwendungszeitbegrenzung erreicht, bis die Verwendungszeit die Verwendungszeitbegrenzung erreicht, ermittelt des Verwendungszustands des Endoskops (10) als gebrauchsabgeschlossen, nachfolgendes Verbieten der Verwendung des Endoskops (10).

3. Steuerungsverfahren für eine Gastrointestinaltraktdiagnosevorrichtung mit einem verfügbaren Endoskop (10) nach Anspruch 1, wobei wenn die Arbeitsstation (30) die gleiche ist, wie die andere Arbeitsstation (30), die das letzte Mal verbunden war, weiter Ermitteln, ob eine Verwendungszeit des Endoskops (10) eine Verwendungszeitbegrenzung erreicht, bis die Verwendungszeit die Verwendungszeitbegrenzung erreicht, Bestimmen des Verwendungszustands des Endoskops (10) als gebrauchsabgeschlossen, nachfolgendes Verbieten der Verwendung des Endoskops (10).

4. Steuerungsverfahren für eine Gastrointestinaltraktdiagnosevorrichtung mit einem verfügbaren Endoskop (10) nach Anspruch 1, wobei eingestellt wird, dass das Endoskop (10) durch eine Verwendungszeitbegrenzung begrenzt ist, die Verwendungszeitbegrenzung einen Anfangswert und einen Endwert aufweist, wobei der Schritt (a) das Einleiten einer Zeitsteuereinheit der Arbeitsstation (30) um einen Zeitzählwert zu erzeugen,
wobei wenn der Zeitzählwert gleich dem Anfangswert ist, Ermitteln des Verwendungszustands des Endoskops (10) als nicht in Gebrauch befindlich,
wenn der Zeitzählwert gleich oder über dem Endwert liegt, Ermittel des Verwendungszustands des Endoskops (10) als gebrauchsabgeschlossen, wenn der Zeitzählwert zwischen dem Anfangswert und dem Endwert liegt,
Ermitteln des Verwendungszustands des Endoskops (10) als in Gebrauch befindlich.

5. Gastrointestinaltraktdiagnosegerät mit einem verfügbaren Endoskop (10) zumindest umfassend:
ein Endoskop (10) mit einem Schaltkreismodul (12), wobei das Schaltkreismodul (12) zumindest ein Substrat (122) und eine auf dem Substrat (122) angeordnete Steuereinheit (1220) aufweist,
ein Bildaufnahmemodul (13), das zumindest eine erste Linse (131) und einen Bildsensor (131a) aufweist, die auf dem Substrat (122) angeordnet sind,
ein Beleuchtungsmodul (14), das zumindest eine erste Lichtquelle (141) aufweist, die auf dem Substrat (122) angeordnet ist,
einen an einer Verbindungseinrichtung (16, 17) montierten Gehäusekörper (15), um das Schaltkreismodul (12), das Bilderfassungsmodul (13) und das Beleuchtungsmodul (14) wasserdicht zu umschließen, und
eine Arbeitsstation (30),
wobei die Arbeitsstation (30) ermittelt, ob ein Verwendungszustand des Endoskops (10) durch einen Zeitgeber (31) überfällig ist,
**dadurch gekennzeichnet, dass**
das Schaltkreismodul (12) einen auf dem Substrat (122) angeordnete Erkennungsmarker (1220') aufweist, und
die Arbeitsstation (30) ausgebildet ist mit dem Endoskop (10) verbunden zu werden, um auf den Erkennungsmarker (1220') zuzugreifen und eine Maschinenidentität in den Erkennungsmarker (1220') hinzuzufügen, um mit dem Endoskop (10) übereinzustimmen, und
die Arbeitsstation (30) ferner ausgebildet ist, zu ermitteln, ob das Endoskop (10) durch den Erkennungsmarker (1220') und die Maschinenidentität mit einer anderen Arbeitsstation (30) verbunden war,
die sich von der Arbeitsstation (30) unterscheidet,
wobei das Endoskop (10) darin eine Magnetplatte (11) aufweist, die Magnetplatte (11) einen ersten Magnetpol (111) und einen zweiten Magnetpol (112) aufweist, die jeweils an einem unteren Halbabschnitt und einem oberen Halbabschnitt der Magnetplatte (11) entlang einer Dickenrichtung (TD) der Magnetplatte (11) angeordnet sind, wobei die Magnetplatte (11) mit dem Schaltkreismodul (12) befestigt ist,
wobei die Gastrointestinaltraktdiagnosevorrichtung mit einem verfügbaren Endoskop (10) ferner
eine magnetische Steuervorrichtung (20) umfasst mit:
einen Magnetstab (21) mit einem ersten elektrischen Magnetpol (211) an einem Anschlußabschnitt des Magnetstabs (21) und einem zweiten elektrischen Magnetpol (212), der an einem Basisabschnitt des Magnetstabs (21) angeordnet ist,
ein magnetisches Kraftausgangsmodul (22), das ausgebildet ist, um einen vorbestimmten Strom an den Magnetstab (21) auszugeben, wobei der Magnetstab (21) dazu ausgelegt ist, eine vorbestimmte magnetische Kraft zum Zusammenwirken mit dem ersten Magnetpol (111) und dem zweiten Magnetpol (112) auszugeben, und
ein Magnetkraftrückkopplungsmodul (23),
wobei die vorbestimmte magnetische Kraft dazu ausgebildet ist, die Magnetplatte (11) zu ziehen, um die Bewegung des Endoskops (10) zu steuern,
wobei die Magnetplatte (11) dazu ausgebildet ist, eine Rückkopplungsmagnetkraft auf einen Sensor des Magnetstabes (21) zu bewirken, Umwandeln des vorbestimmten Stroms in einen Offsetstrom, und wobei das Magnetkraftrückkopplungsmodul (23) angepasst ist, den Offsetstrom so zu kalibrieren, dass der Offsetstrom wieder auf den vorbestimmten Strom zurückführt wird, um so die vorbestimmte magnetische Kraft zu stabilisieren.

6. Gastrointestinaltraktdiagnosegerät mit einem verfügbaren Endoskop (10) nach Anspruch 5, wobei die Verbindungsvorrichtung (16, 17) ferner eine leitfähige Schnittstelle (16a) und eine Übertragungsleitung (160) umfasst, wobei der eine Anschluss der Übertragungsleitung mit dem Schaltkreismodul durch die leitfähige Schnittstelle verbunden ist,
der andere Anschluss der Übertragungsleitung (170) aus der Verbindungseinrichtung (17) herausragt, um mit der Arbeitsstation (30) zu verbunden zu sein.

7. Gastrointestinaltraktdiagnosegerät mit einem verfügbaren Endoskop (10) nach Anspruch 5, wobei sich ein Halter (1221) schräg von einem freien Ende des Substrats (122) erstreckt und die erste Linse (131) auf dem Halter (1221) angeordnet ist.

8. Gastrointestinaltraktdiagnosegerät mit einem verfügbaren Endoskop (10) nach Anspruch 5, wobei das Bildaufnahmemodul (13) eine zweite Linse (132) aufweist, wobei die zweite Linse (132) auf einer Oberfläche (122a) des Substrats angeordnet ist (122).

9. Gastrointestinaltraktdiagnosegerät mit einem verfügbaren Endoskop (10) nach Anspruch 5, wobei der Gehäusekörper (15) einen ersten polierten Bereich (151), einen zweiten polierten Bereich (152) und einen darauf befindlichen matten gleichförmigen Bereich (153) aufweist, wobei der erste polierte Bereich (151) der ersten Linse (131) zugeordnet ist und der zweite polierte Bereich (152) der zweiten Linse (132) zugeordnet ist.

10. Gastrointestinaltraktdiagnosevorrichtung mit einem verfügbaren Endoskop (10) nach Anspruch 5, wobei die magnetische Steuervorrichtung (20) einen Halteabschnitt (24), der mit dem Basisabschnitt des Magnetstabs (21) verbunden ist aufweist, wobei der Halteabschnitt (24) aufweist:
eine erste Seite (241) mit einer ersten Boottaste (C1), die in einen ausgeschalteten Zustand voreingestellt ist,
eine zweite Seite (242) mit einer zweiten Boottaste (C2), die in einen ausgeschalteten Zustand voreingestellt ist,
einen Bauchabschnitt (243) mit einem darauf befindlichen Ring (C3) zum Steuern eines Spinzustands des Magnetstabs (21), und
einen Rückenabschnitt (244) mit Manipulationstasten (C4) und einen Bildschirm (C5) darauf, wobei die erste Boottaste (C1) und die zweite Boottaste (C2) zunächst gedrückt werden, um den ausgeschalteten Zustand des Rings (C3) und der Manipulationstasten zu entriegeln (C4).

11. Gastrointestinaltraktdiagnosevorrichtung mit einem verfügbaren Endoskop (10) nach Anspruch 10, wobei die Manipulationstasten (C4) Menütasten (C43), eine erste Taste (C41) und eine zweite Taste (C42) umfassen, wobei die erste Taste (C41) an einer ersten Stelle (2441) des hinteren Teils (244) angeordnet ist, um von einem Daumen einer rechten Hand gedrückt zu werden,
die zweite Taste (C42) an einer zweiten Stelle (2442) des hinteren Teils (244) angeordnet ist, um durch einen Daumen einer linken Hand gedrückt zu werden, und
die Menütasten (C43) an einer dritten Stelle (2443) des hinteren Teils (244) angeordnet sind, um durch den Daumen der linken Hand oder der rechten Hand gedrückt zu werden.

12. Gastrointestinaltraktdiagnosevorrichtung mit einem verfügbaren Endoskop (10) nach Anspruch 10, wobei der Bauchabschnitt (243) und der Magnetstab (21) einen Winkel zwischen 155 und 175 Grad ausbilden.

13. Gastrointestinaltraktdiagnosevorrichtung mit einem verfügbaren Endoskop (10) nach Anspruch 11, wobei der Bauchabschnitt (243) und der Magnetstab (21) einen Winkel zwischen 155 und 175 Grad ausbilden.

14. Gastrointestinaltraktdiagnosevorrichtung mit einem verfügbaren Endoskop (10) nach Anspruch 11, wobei die erste Taste (C41) und die zweite Taste (C42) jeweils eine erste Taste eines zweistufigen Drückens und eine zweite Taste des zweistufigen Drückens sind.

## Revendications

1. Procédé de commande d'un dispositif de diagnostic du tractus gastro-intestinal avec un endoscope jetable (10), comprenant au moins les étapes suivantes consistant à :
(a) fournir un endoscope (10), et connecter de manière électrique l'endoscope (10) à une station de travail (30) ;
(b) déterminer l'état d'utilisation de l'endoscope (10) ; et
(c) déterminer l'endoscope (10) comme étant non utilisé, en service, ou comme ayant été utilisé, selon l'état d'utilisation de l'endoscope (10), dans lequel :
si l'état d'utilisation de l'endoscope (10) est déterminé comme ayant été utilisé, interdire l'utilisation de l'endoscope (10) ;
**caractérisé en ce que**, si l'état d'utilisation de l'endoscope (10) est déterminé comme étant en service, continuer de déterminer si la station de travail (30) à laquelle est connecté l'endoscope (10), est identique à une autre station de travail (30) à laquelle a été connecté l'endoscope (10) la dernière fois, dans lequel si l'ID machine de la station de travail (30) est différent de l'ID machine de l'autre station de travail (30) connectée la dernière fois, interdire l'utilisation de l'endoscope (10).

2. Procédé de commande d'un dispositif de diagnostic du tractus gastro-intestinal avec un endoscope jetable (10) selon la revendication 1, dans lequel, si l'état d'utilisation de l'endoscope (10) est déterminé comme étant non utilisé, continuer de déterminer si le temps d'utilisation atteint une limite de temps d'utilisation jusqu'à ce que le temps d'utilisation atteigne la limite de temps d'utilisation ; déterminer l'état d'utilisation de l'endoscope (10) comme ayant été utilisé, interdire par la suite l'utilisation de l'endoscope (10).

3. Procédé de commande d'un dispositif de diagnostic du tractus gastro-intestinal avec un endoscope jetable (10) selon la revendication 1, dans lequel, si la station de travail (30) est identique à l'autre station de travail (30) connectée la dernière fois, continuer de déterminer si le temps d'utilisation de l'endoscope (10) atteint une limite de temps d'utilisation jusqu'à ce que le temps d'utilisation atteigne la limite de temps d'utilisation ; déterminer l'état d'utilisation de l'endoscope (10) comme ayant été utilisé, interdire par la suite l'utilisation de l'endoscope (10).

4. Procédé de commande d'un dispositif de diagnostic du tractus gastro-intestinal avec un endoscope jetable (10) selon la revendication 1, dans lequel l'endoscope (10) est réglé de façon à être limité par une limite de temps d'utilisation, la limite de temps d'utilisation présentant une valeur initiale et une valeur finale, l'étape (a) comprenant une étape consistant à déclencher une unité de synchronisation de la station de travail (30) de manière à générer une valeur de comptage de synchronisation ;
dans lequel lorsque la valeur de comptage de synchronisation est égale à la valeur initiale, déterminer l'état d'utilisation de l'endoscope (10) comme étant non utilisé ;
dans lequel lorsque la valeur de comptage de synchronisation est égale ou supérieure à la valeur finale, déterminer l'état d'utilisation de l'endoscope (10) comme ayant été utilisé ;
dans lequel lorsque la valeur de comptage de synchronisation est comprise entre la valeur initiale et la valeur finale, déterminer l'état d'utilisation de l'endoscope (10) comme étant en service.

5. Dispositif de diagnostic du tractus gastro-intestinal avec un endoscope jetable (10), comprenant au moins :
un endoscope (19) qui présente un module de circuit (12), le module de circuit (12) comprenant au moins un substrat (122) et une unité de commande (1220) qui est disposée sur le substrat (122) ;
un module d'acquisition d'image (13) qui comprend au moins une première lentille (131) et un capteur d'image (131a) qui sont disposés sur le substrat (122) ;
un module d'éclairage (14) qui comprend au moins une première source de lumière (141) disposée sur le substrat (122) ;
un corps formant enceinte (15) raccordé à un dispositif de connexion (16 ou 17), destiné à enfermer de manière étanche à l'eau le module de circuit (12), le module d'acquisition d'image(13) et le module d'éclairage (14) ; et
une station de travail (30) ;
dans lequel la station de travail (30) détermine si l'état d'utilisation de l'endoscope (10) est non retourné par un temporisateur (31) ;
**caractérisé en ce que** le module de circuit (12) comprend un repère de reconnaissance (1220') qui est disposé sur le substrat (122) ; et
**en ce que** la station de travail (30) est adaptée de façon à être connectée à l'endoscope (10), de manière à accéder au repère de reconnaissance (1220') et à ajouter une identité de la machine dans le repère de reconnaissance (1220') de façon à correspondre à l'endoscope (10) ; et
**en ce que** la station de travail (30) est adaptée en outre de façon à déterminer si l'endoscope (10) a été connecté à une autre station de travail (30) différente de la station de travail (30), grâce au repère de reconnaissance (1220') et à l'identité de la machine ;
dans lequel l'endoscope (10) comprend à l'intérieur une plaque magnétique (11), la plaque magnétique (11) présentant un premier pôle magnétique (111) et un second pôle magnétique (112) répartis respectivement au niveau de la demi-partie inférieure et au niveau de la demi-partie supérieure de la plaque magnétique (11) le long de la direction de l'épaisseur (TD) de la plaque magnétique (11), la plaque magnétique (11) étant fixée avec le module de circuit (12) ;
dans lequel le dispositif de diagnostic du tractus gastro-intestinal avec un endoscope jetable (10) comprend en outre un dispositif de commande magnétique (20) qui comprend :
un bâtonnet magnétique (21) qui comprend un premier pôle magnétique électrique (211) au niveau de la partie terminale du bâtonnet magnétique (21) et un second pôle magnétique électrique (212) situé au niveau de la partie base du bâtonnet magnétique (21) ;
un module génération de force magnétique (22) adapté de façon à délivrer en sortie un courant prédéterminé au bâtonnet magnétique (21), le bâtonnet magnétique (21) étant adapté de manière à générer une force magnétique prédéterminée destinée à interagir avec le premier pôle magnétique (111) et le second pôle magnétique (112) ; et
un module de rétroaction de force magnétique (23) ;
dans lequel la force magnétique prédéterminée est adaptée de façon à attirer la plaque magnétique (11) de manière à commander le déplacement de l'endoscope (10) ;
dans lequel la plaque magnétique (11) est adaptée de façon à provoquer une force magnétique de rétroaction sur un capteur du bâtonnet magnétique (21), à convertir le courant prédéterminé en un courant de décalage, et dans lequel le module de rétroaction de force magnétique (23) est adapté de façon à étalonner le courant de décalage en reprenant le courant de décalage vers le courant prédéterminé, de manière à stabiliser la force magnétique prédéterminée.

6. Dispositif de diagnostic du tractus gastro-intestinal avec un endoscope jetable (10) selon la revendication 5, dans lequel le dispositif de connexion (16 ou 17) comprend en outre une interface conductrice (16a) et une ligne de transmission (160), dans lequel une borne de la ligne de transmission (160) est connectée au module de circuit (12) par l'intermédiaire de l'interface conductrice ;
dans lequel l'autre borne de la ligne de transmission (170) s'étend hors du dispositif de connexion (17) pour être connectée à la station de travail (30).

7. Dispositif de diagnostic du tractus gastro-intestinal avec un endoscope jetable (10) selon la revendication 5, dans lequel un support (1221) s'étend de manière inclinée à partir d'une extrémité libre du substrat (122), et la première lentille (131) est disposée sur le support (1221).

8. Dispositif de diagnostic du tractus gastro-intestinal avec un endoscope jetable (10) selon la revendication 5, dans lequel le module d'acquisition d'image (13) comprend une seconde lentille (132), la seconde lentille (132) étant disposée sur une surface (122a) du substrat (122).

9. Dispositif de diagnostic du tractus gastro-intestinal avec un endoscope jetable (10) selon la revendication 5, dans lequel le corps formant enceinte (15) comprend dessus une première région polie (151), une seconde région polie (152) et une région uniforme mate (153), dans lequel la première région polie (151) correspond à la première lentille (131), et la seconde polie région (152) correspond à la seconde lentille (132).

10. Dispositif de diagnostic du tractus gastro-intestinal avec un endoscope jetable (10) selon la revendication 5, dans lequel le dispositif de commande magnétique (20) comprend : une partie support (24) connectée à la partie base du bâtonnet magnétique (21), la partie support (24) comprenant :
un premier côté (241) avec un premier bouton d'initialisation (C1) préréglé dans un état bloqué par défaut ;
un second côté (242) avec un second bouton d'initialisation (C2) préréglé dans un état bloqué par défaut ;
une partie renflée (243) avec un anneau (C3) dessus, destinée à commander l'état de rotation du bâtonnet magnétique (21) ; et
une partie arrière (244) avec des boutons de manipulation (C4) et un écran (C5) situé dessus, dans lequel le premier bouton d'initialisation (C1) et le second bouton d'initialisation (C2) doivent d'abord être pressés de façon à déverrouiller respectivement l'état bloqué par défaut de l'anneau (C3) et des boutons de manipulation (C4).

11. Dispositif de diagnostic du tractus gastro-intestinal avec un endoscope jetable (10) selon la revendication 10, dans lequel les boutons de manipulation (C4) comprennent des boutons de menu (C43), une première touche (C41) et une seconde touche (C42) ;
dans lequel la première touche (C41) est agencée à un premier emplacement (2441) de la partie arrière (244), de façon à pouvoir être pressée par le pouce de la main droite ;
dans lequel la seconde touche (C42) est agencée à un deuxième emplacement (2442) de la partie arrière (244), de façon à pouvoir être pressée par le pouce de la main gauche ; et
dans lequel les boutons de menu (C43) sont agencés à un troisième emplacement (2443) de la partie arrière (244), de façon à pouvoir être pressés par le pouce de la main droite ou par celui de la main gauche.

12. Dispositif de diagnostic du tractus gastro-intestinal avec un endoscope jetable (10) selon la revendication 10, dans lequel la partie renflée (243) et le bâtonnet magnétique (21) font un angle compris entre 155 degrés et 175 degrés.

13. Dispositif de diagnostic du tractus gastro-intestinal avec un endoscope jetable (10) selon la revendication 11, dans lequel la partie renflée (243) et le bâtonnet magnétique (21) font un angle compris entre 155 degrés et 175 degrés.

14. Dispositif de diagnostic du tractus gastro-intestinal avec un endoscope jetable (10) selon la revendication 11, dans lequel la première touche (C41) et la seconde touche (C42) sont respectivement la première touche d'un poussoir à deux étages, et la seconde touche d'un poussoir à deux étages.
